# FASCICULE DE BREVET EUROPEEN

(11) **EP 3 881 007 B1**
(45) Date de publication et mention de la délivrance du brevet: **09.07.2025**
(21) Numéro de dépôt: 19801058.9
(22) Date de dépôt: 14.11.2019
(51) Int. Cl.: F24C 7/08, G01N 33/00, A47J 27/08

(54) **DISPOSITIF AMOVIBLE POUR ANALYSER LES ODEURS GÉNERÉES DANS UN APPAREIL ÉLECTROMÉNAGER, PROCÉDÉ DE CALIBRATION DU DISPOSITIF AMOVIBLE, ET PROCÉDÉ DE CUISSON POUR UN APPAREIL ÉLECTROMENAGER ÉQUIPE DU DISPOSITIF AMOVIBLE**
ABNEHMBARE VORRICHTUNG ZUM ANALYSIEREN DER IN EINEM ELEKTRISCHEN HAUSHALTSGERÄT ERZEUGTEN GERÜCHE, VERFAHREN ZUM KALIBRIEREN DER ABNEHMBAREN VORRICHTUNG UND KOCHVERFAHREN FÜR EIN ELEKTRISCHES HAUSHALTSGERÄT MIT DER ABNEHMBAREN VORRICHTUNG
REMOVABLE DEVICE FOR ANALYSING THE ODOURS PRODUCED IN AN ELECTRICAL HOUSEHOLD APPLIANCE, METHOD FOR CALIBRATING THE REMOVABLE DEVICE, AND COOKING METHOD FOR AN ELECTRICAL HOUSEHOLD APPLIANCE EQUIPPED WITH THE REMOVABLE DEVICE

(30) Priorité: 15.11.2018 FR 1871544
(43) Date de publication de la demande: 22.09.2021
(73) Titulaire: SEB S.A., 69130 Ecully (FR)
(72) Inventeur: GAUTHIER, Sébastian, 69007 LYON (FR); GAILHARD, Thierry, 69970 CHAPONNAY (FR)
(74) Mandataire: Germain Maureau
(86) Numéro de dépôt international: PCT/EP2019/081415
(87) Numéro de publication internationale: WO 2020/099608

(56) Documents cités:
- EP-A1- 1 437 925
- DE-A1- 102007 054 340
- FR-A1- 2 451 145
- JP-A- S62 129 620
- US-A1- 2017 127 700

## Description

La présente invention concerne le domaine des équipements d'aide à la cuisson des aliments, et concerne en particulier l'application d'un dispositif de détection d'odeurs à un appareil électroménager de cuisson.

Il est connu d'utiliser un capteur de gaz, de type nez électronique, placé à l'intérieur d'un four pour réagir à des composés organiques volatils (COV) émis pendant une cuisson. Il est également connu, par exemple du document US1024477882 de placer un tel capteur à l'extérieur de l'enceinte de cuisson du four. Dans ces configurations, le module embarqué de détection des odeurs des gaz a vocation à faire partie intégrante de l'appareil de cuisson, le four. Or, les capteurs de gaz sont très sensibles aux conditions d'environnement, telles que température, humidité, environnement électromagnétique, etc..., et nécessitent une calibration, rendue complexe lorsque le dispositif se trouve à l'intérieur de l'appareil et en fait intégralement partie. En outre, l'environnement mécanique à l'intérieur du four est contraignant du fait des vibrations et des déformations subies par les parois internes du four. Enfin la maintenance et/ou le remplacement des parties consommables est difficile à envisager, dans ces configurations, du fait des contraintes d'accès à l'intérieur du four.

Il est également connu de disposer de capteur de gaz, de type nez électronique, de manière autonome. Cependant, un tel type de capteur est très dépendant du placement libre de l'utilisateur, la mesure des taux des odeurs significatives est faible, la perturbation des odeurs parasites est peu maitrisée, il existe un risque de mauvaise utilisation qui fausse la mesure voire détruit le capteur, la calibration est aléatoire et trop dépendante du milieu à mesurer et enfin un tel capteur est coûteux du fait de sa consommation d'énergie et de l'autonomie souhaitée.

L'invention a donc pour but de proposer une solution à tout ou partie de ces problèmes.

A cet effet, la présente invention concerne un module amovible pour analyser les odeurs générées dans une enceinte de cuisson d'un appareil électroménager, selon la revendication 1.

Selon ces dispositions, le module amovible est positionné dans une situation étudiée, facilement accessible par l'utilisateur, et où l'environnement est stabilisé et les conditions de mesure sont adaptées.

Selon ces dispositions, la mise en œuvre du module amovible par le consommateur est simplifiée, notamment pour la mise en œuvre de sa calibration, et pour l'usage d'éléments consommables à l'intérieur du boitier du module amovible dans le cadre de la maintenance du module amovible.

Selon un mode de réalisation, les gaz odoriférants émis par l'appareil au cours d'une cuisson sont canalisés, filtrés, pré-concentrés, ou épurés, dans la portion de l'enceinte de cuisson de l'appareil électroménager qui est en communication gazeuse avec le module amovible, pour focaliser la mesure par l'élément sensible sur une caractéristique prédéterminée.

Selon un mode de réalisation, l'élément sensible comprend un ou plusieurs composants parmi la liste ci-après : un composant du type micro-balance à quartz fonctionnalisé, un composant du type capteur de gaz à oxydes métalliques, un composant du type capteur de gaz électrochimique, un composant du type détecteur à photo-ionisation pour l'analyse et la quantification des composés organiques volatiles, ou un composant du type à prisme fonctionnalisé ce dernier type comprenant notamment une source optique et un capteur CCD.

Selon un mode de réalisation, la partie mécanique est configurée pour assurer l'étanchéité aux fluides non gazeux de l'interface mécanique entre l'appareil électroménager et le module fixé à la paroi de l'appareil électroménager de manière amovible et pour filtrer les gaz entrants dans le boitier en provenance de l'enceinte de cuisson de l'appareil électroménager de manière à réduire l'humidité relative et le taux de matières grasses à l'état de vapeur.

L'invention concerne également un appareil électroménager équipé d'un module amovible selon l'invention, l'appareil électroménager comprenant les moyens de fixation complémentaires agencés sur la surface externe de la paroi de l'appareil électroménager pour interagir avec les moyens de fixation du module amovible et fixer de manière amovible sur l'appareil électroménager le module amovible dans une position fonctionnelle dans laquelle la conduite d'entrée du boitier du module amovible est en communication gazeuse avec un passage ménagé au travers de la paroi de l'appareil électroménager et débouchant dans une portion de l'enceinte de cuisson de l'appareil électroménager.

Selon un mode de réalisation, l'appareil électroménager comprend une ou plusieurs des caractéristiques suivantes, seules ou en combinaison.

Selon un mode de réalisation, l'appareil électroménager comprend un élément complémentaire d'alimentation électrique et de communication de données, l'élément complémentaire étant complémentaire de l'élément d'alimentation électrique et de l'élément de communication de données du module amovible, l'élément complémentaire étant configuré pour raccorder électriquement l'élément d'alimentation électrique du module amovible à une alimentation électrique l'appareil électroménager et pour assurer une communication de données entre l'élément de communication de données du module amovible et une unité de contrôle et de régulation de l'appareil électroménager.

Selon un mode de réalisation, l'appareil électroménager est un four, de préférence un four à chaleur tournante.

Selon un mode de réalisation, l'appareil électroménager est un cuiseur électrique sous pression.

Selon un mode de réalisation, la transmission d'énergie électrique entre l'élément complémentaire et l'élément d'alimentation électrique du module amovible est réalisée sans fil.

Selon un mode de réalisation, la communication de données entre l'élément complémentaire et l'élément de communication de données du module amovible est réalisée sans fil.

Selon un aspect, l'invention concerne un procédé de cuisson d'un plat avec un appareil électroménager selon l'invention, comprenant les étapes suivantes :
- Sélection par un utilisateur d'un plat via une interface utilisateur, l'unité de contrôle et de régulation de l'appareil électroménager étant configurée pour communiquer avec une base de données comportant une liste de plats et des programmes de cuisson associés audits plats, lesdits programmes de cuisson comportant une température de régulation, un temps de cuisson et au moins une valeur de dépassement de seuils de teneurs en composés organiques volatiles,
- Commande d'une séquence de cuisson dudit plat comprenant une succession de couples de température de régulation et de temps de cuisson, la commande étant réalisée par l'unité de contrôle et de régulation de l'appareil électroménager, la température et/ou le temps de cuisson étant ajustés en cours de cuisson sur la base d'une détection d'une valeur de dépassement de seuils de teneurs en composés organiques volatiles détectée par le module amovible en cours de cuisson du plat dans l'enceinte de cuisson de l'appareil électroménager.

Selon un mode de mise en œuvre du procédé de cuisson, la détection d'une valeur de dépassement de seuil de teneurs en composés organiques volatiles est une moyenne de valeurs de dépassement de seuils de teneurs en composés organiques volatiles détectées sur une période de temps.

Selon un mode de mise en œuvre, l'appareil électroménager est configuré pour fonctionner selon un mode de cuisson intelligente dans lequel le module amovible notifie périodiquement ou lors d'un dépassement de seuil, au cours de la cuisson, à l'unité de contrôle et de régulation de l'appareil électroménager un temps de cuisson restant et/ou une température de régulation en fonction des réactions détectées par l'élément sensible du module amovible, l'élément sensible étant sensible aux composés organiques volatiles émis dans l'enceinte de cuisson de l'appareil électroménager pendant la cuisson, les composés organiques volatiles émis étant caractéristiques de l'évolution de la cuisson pour une recette donnée.

Selon un aspect, l'invention concerne également un procédé de calibration d'un module amovible selon l'invention pour analyser les odeurs générées dans une enceinte de cuisson de l'appareil électroménager, le procédé comprenant les étapes suivantes :
- Sélection via l'interface utilisateur d'un programme de calibration;
- Commande, par l'unité de contrôle et de régulation de l'appareil électroménager, du chauffage de l'enceinte de cuisson de l'appareil électroménager à une température prédéterminée;
- Lorsque la température prédéterminée est atteinte dans l'enceinte de cuisson, positionnement du module amovible sur l'appareil électroménager;
- Calibration de l'élément sensible du module amovible sur la base d'un dépassement de seuil de teneurs en composés organiques volatiles présents dans l'enceinte de cuisson.

Selon un mode de mise en œuvre du procédé de calibration, la température prédéterminée est d'environ 180°C.

Selon une mise en œuvre du procédé, le programme de calibration comprend une étape de préchauffage de l'appareil électroménager.

Pour sa bonne compréhension, l'invention est décrite en référence aux dessins ci-annexés représentant, à titre d'exemple non limitatif, une forme de réalisation d'un dispositif selon l'invention. Les mêmes références sur les dessins désignent des éléments similaires ou des éléments dont les fonctions sont similaires.
La figure 1 est une illustration schématique d'un appareil électroménager de cuisson équipé du dispositif selon un mode de réalisation de l'invention.
La figure 2 est une illustration schématique du dispositif selon un mode de réalisation de l'invention.
La figure 3 est une illustration schématique d'un procédé de cuisson selon l'invention.
La figure 4 est une illustration schématique d'un procédé de calibration du dispositif selon l'invention.

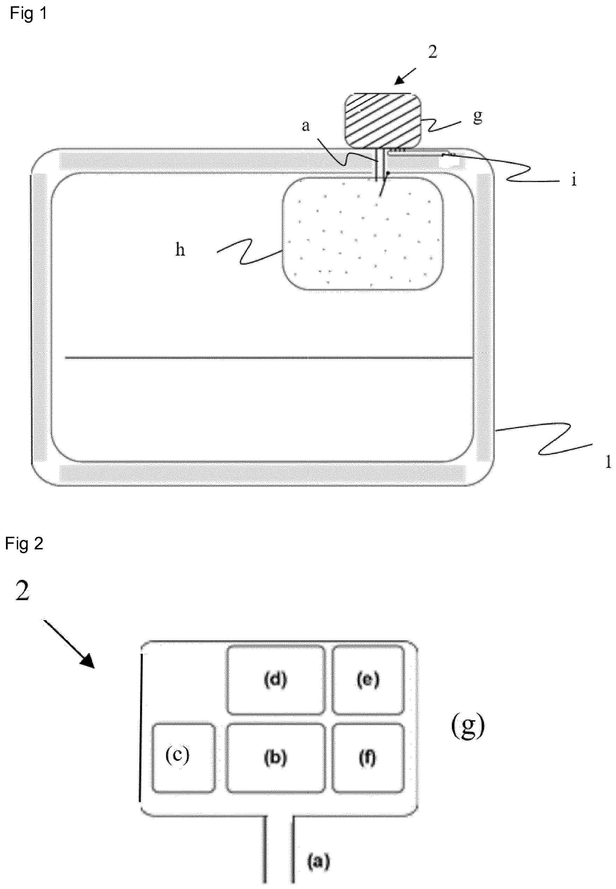

Selon un mode de réalisation illustré par la figure 1, l'appareil électroménager est un four 1 équipé d'un dispositif agencé pour réagir, pendant la cuisson d'un plat, aux odeurs générées à l'intérieur de l'enceinte de cuisson du four, sous la forme de composés organiques volatiles; les mesures ainsi réalisées permettent notamment de caractériser l'avancement de la cuisson dudit plat, et de déterminer une température de régulation et/ou un temps de cuisson restant, et permettent ainsi de mettre en œuvre un procédé de cuisson intelligent, selon un aspect de l'invention. Selon un mode de réalisation, le four est un four à chaleur tournante, i.e. à convection, configuré pour recueillir les odeurs émises pendant la cuisson et les canaliser vers une portion h du volume intérieur de l'enceinte de cuisson du four 1. Ladite portion h est, selon un mode de réalisation, une zone de surpression liée à la convection forcée dans le four.

Le dispositif agencé pour réagir aux odeurs est un module g, positionné de manière amovible à l'extérieur du four 1, par exemple sur une surface externe d'une paroi du four 1, de manière à ce qu'il soit facilement accessible par un utilisateur du four 1.

Le module g amovible comprend un boitier équipé d'une conduite d'entrée a, la conduite d'entrée a débouchant à l'intérieur du boitier par une extrémité ; par une autre extrémité, lorsque le module g amovible est positionné sur la surface externe de la paroi du four 1, la conduite d'entrée est en communication gazeuse avec un passage ménagé au travers de la paroi du four et débouchant dans la portion h de l'enceinte de cuisson du four 1. Ainsi les composés organiques volatiles générés pendant une cuisson à l'intérieur de l'enceinte de cuisson du four, selon un mode de réalisation, dans ladite portion h de l'enceinte de cuisson du four, sont naturellement poussés de l'enceinte de cuisson vers l'intérieur du boitier du module g amovible, lorsque celui-ci est positionné sur la surface externe de la paroi du four 1.

Le boitier défini un espace confiné. Ce boitier n'est donc pas placé dans un chemin d'écoulement des gaz provenant de l'enceinte de cuisson vers l'extérieur.

Le module g amovible comprend des moyens de fixation destinés à interagir avec des moyens de fixation complémentaires agencés sur la surface externe de la paroi du four 1, de manière à fixer de manière amovible le module g amovible relativement à la paroi du four dans une position fonctionnelle dans laquelle la conduite d'entrée du boitier est en communication gazeuse avec le passage ménagé au travers de la paroi du four, débouchant dans la portion h de l'enceinte de cuisson du four 1.

Selon le mode de réalisation, illustré sur la figure 2, le module g amovible comprend, à l'intérieur du boitier :
- un élément b sensible aux gaz, qui est agencé pour réagir à des dépassements de seuils de teneurs en composés organiques volatiles des gaz odoriférants qui passent de l'enceinte de cuisson du four jusqu'à l'intérieur du boîtier;
- un élément c d'aspiration, qui est agencé pour favoriser, en les aspirant, le prélèvement des gaz odoriférants depuis l'enceinte de cuisson du four 1, jusqu'à l'intérieur du boitier, en passant par la conduite d'entrée a du boitier,
- un élément d de chauffage, qui est agencé pour chauffer l'intérieur du boitier afin d'éviter la condensation dans le boitier des gaz chauds et humides en provenance de l'enceinte de cuisson et assurer une température stable de fonctionnement au niveau de l'élément sensible;
- un élément e d'alimentation électrique, configuré pour être raccordé électriquement à une source d'énergie électrique externe au boitier,
- un élément f de communication de données.

L'élément b sensible aux gaz comprend un élément configuré pour détecter la présence de certaines molécules gazeuses : à cet effet, l'élément b sensible aux gaz comprend un un ou plusieurs composants parmi la liste ci-après : un composant du type micro-balance à quartz fonctionnalisé, composant du type capteur de gaz à oxydes métalliques, composant du type capteur de gaz électrochimique, composant du type détecteur à photo-ionisation pour l'analyse et la quantification des composés organiques volatiles, un composant du type à prisme fonctionnalisé ce dernier type comprenant notamment une source optique et un capteur CCD.

L'espace confiné défini par le boitier permet de contrôler l'environnement de l'élément sensible aux gaz.

Les composés organiques volatiles émis par l'appareil au cours d'une cuisson sont, par exemple, canalisés, filtrés, pré-concentrés, ou épurés, dans la portion de l'enceinte du four qui est en communication gazeuse avec le module amovible, pour focaliser la mesure par l'élément sensible sur une caractéristique prédéterminée.

L'interface mécanique qui assure le couplage mécanique entre le module g amovible et le four 1 est configurée pour être étanche aux fluides non gazeux, lorsque le module g amovible est fixé à la paroi du four 1, et pour filtrer les gaz entrants dans le boitier, en provenance du four, de manière à réduire l'humidité relative et le taux de matières grasses à l'état de vapeur.

Le four 1 comprend une unité de contrôle et de régulation du four 1, non représentée sur la figure 1.

Le four 1 comprend un élément complémentaire i d'alimentation électrique et de communication de données, l'élément complémentaire i étant configuré pour raccorder électriquement l'élément e d'alimentation électrique du module g à une alimentation électrique du four 1 et pour assurer une communication de données entre l'élément f de communication de données du module g et l'unité de contrôle et de régulation du four 1, lorsque le module g est fixé, de manière amovible, à la paroi du four 1. Selon ces dispositions, lorsque le module g est fixé, de manière amovible, à la paroi du four 1, il est alimenté électriquement, et il communique les données détectées par l'élément b sensible du module, relatives aux composés organiques volatiles, à l'unité de contrôle et de régulation du four 1; lorsque le module g est fixé, de manière amovible, à la paroi du four 1, il peut également recevoir des commandes d'activation en provenance de l'unité de contrôle et de régulation du four 1, notamment pour la calibration de l'élément b sensible aux gaz du module g.

Le four 1 comprend une interface utilisateur, non représentée sur la figure, configurée pour communiquer avec l'unité de contrôle et de régulation du four 1, cette unité de contrôle et de régulation étant configurée pour communiquer avec une base de données comportant une liste de plats et des programmes de cuisson associés audits plats, lesdits programmes de cuisson comportant une température de régulation, un temps de cuisson et au moins une valeur de dépassement de seuil de teneurs en composés organiques volatiles.

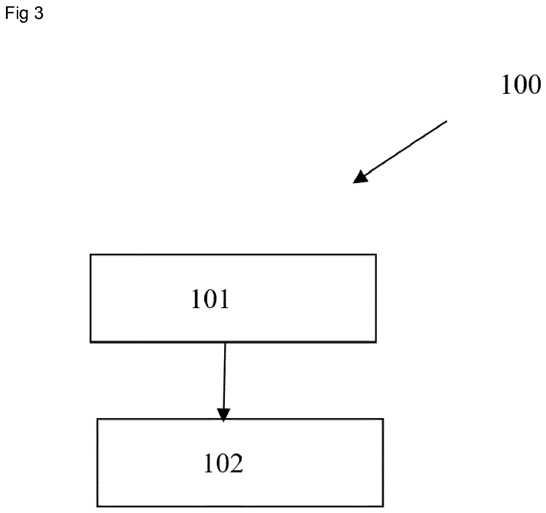

Selon ces dispositions, la mise en œuvre d'un procédé 100 de cuisson intelligent est possible. Selon un mode de mise en œuvre, illustré par la figure 3, le procédé 100 de cuisson comprend les étapes suivantes :
- Sélection 101 par un utilisateur d'un plat, via l'interface utilisateur;
- Commande 102 d'une température de régulation et d'un temps de cuisson définis par un programme de cuisson associé dans la base de données audit plat, la commande étant réalisée par l'unité de contrôle et de régulation du four 1, la température et/ou le temps de cuisson étant ajustés en cours de cuisson sur la base d'une détection d'une valeur de dépassement de seuils de teneurs en composés organiques volatiles détectée par le module g amovible en cours de cuisson dudit plat.

La détection du dépassement de seuils de teneurs en composés organiques volatiles peut par exemple être une moyenne d'un nombre de dépassement de seuils de teneurs en composés organiques volatiles détectées sur une période de temps.

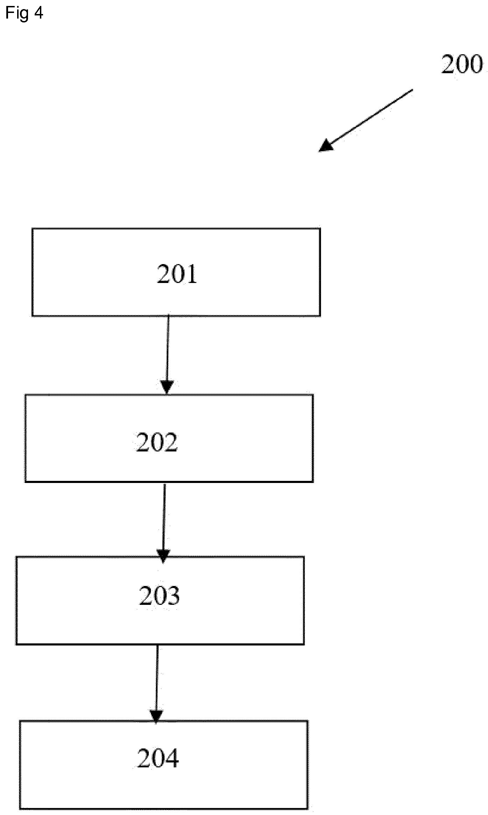

L'invention concerne également un procédé 200 de calibration du module g amovible pour analyser les odeurs générées dans une enceinte de cuisson d'un four 1, le procédé comprenant les étapes suivantes, illustrées sur la figure 4 :
- Sélection 201 via l'interface utilisateur d'un programme de calibration;
- Commande 202, par l'unité de contrôle et de régulation du four 1, d'une séquence de chauffage de l'enceinte de cuisson du four 1 à une température prédéterminée;
- Lorsque la température prédéterminée est atteinte dans l'enceinte de cuisson, positionnement 203 du module g amovible sur le four 1;
- Calibration 204 de l'élément b sensible du module g amovible sur la base du dépassement de seuils de teneurs en composés organiques volatiles présents dans l'enceinte de cuisson;

La température prédéterminée est par exemple d'environ 180°C.

Dans un autre mode de réalisation dans lequel l'appareil électroménager serait un cuiseur électrique sous pression, le couplage mécanique entre la paroi du cuiseur électrique sous pression et le module amovible (g) sera situé au niveau de la soupape de libération de vapeur qui est actionnée électriquement par le produit.

Ce dispositif de coulage permettra de diviser le flux de vapeur sortante : la majorité du flux de vapeur sera dirigée vers l'extérieur, et seulement une portion du flux de vapeur sera dirigée vers le module amovible (g).

Le chemin amenant l'odeur vers le capteur sera construit de sorte que la température du gaz diminue et qu'une partie de l'humidité se condense sur des surfaces découplées thermiquement et à température ambiante.

De plus, le module amovible (g) communique avec le cuiseur électrique sous pression pour réaliser l'acquisition d'une nouvelle mesure d'odeur. En conséquence, le cuiseur électrique sous pression active la soupape de remise à l'air de manière à alimenter en odeur le module amovible (g).

Bien que l'invention ait été décrite en liaison avec des exemples particuliers de réalisation et d'application, il est bien évident qu'elle n'y est nullement limitée.

## Revendications

1. Module (g) amovible pour analyser les odeurs générées dans une enceinte de cuisson d'un appareil électroménager (1), le module (g) comprenant un boitier équipé d'une conduite d'entrée (a), la conduite d'entrée (a) débouchant à l'intérieur du boitier, le module (g) comprenant, à l'intérieur du boitier :
- au moins un élément (b) sensible aux gaz agencé pour réagir à des dépassements de seuils de teneurs en composés organiques volatiles;
- un élément (c) d'aspiration agencé pour prélever un gaz depuis l'extérieur de la conduite d'entrée du boitier vers l'intérieur du boitier ;
- un élément (d) de chauffage ;
- un élément (e) d'alimentation électrique, configuré pour être raccordé électriquement à une source d'énergie électrique externe au boitier,
- un élément (f) de communication de données;
le module (g) amovible étant positionné de manière à ce qu'il soit facilement accessible par un utilisateur du four (1), et comprenant des moyens de fixation destinés à interagir avec des moyens de fixation complémentaires agencés sur une surface externe d'une paroi de l'appareil électroménager de manière à fixer de manière amovible le module amovible relativement à la paroi de l'appareil électroménager dans une position fonctionnelle dans laquelle la conduite d'entrée du boitier est en communication gazeuse avec un passage ménagé au travers de la paroi de l'appareil électroménager et débouchant dans une portion (h) de l'enceinte de cuisson de l'appareil électroménager (1), le boitier définissant un espace confiné.

2. Appareil électroménager (1) équipé d'un module amovible (g) selon la revendication 1, le four (1) comprenant les moyens de fixation complémentaires agencés sur la surface externe de la paroi du four (1) pour interagir avec les moyens de fixation du module (g) amovible et fixer de manière amovible sur le four (1) le module (g) amovible dans une position fonctionnelle dans laquelle la conduite d'entrée du boitier du module (g) amovible est en communication gazeuse avec un passage ménagé au travers de la paroi du four et débouchant dans une portion de l'enceinte de cuisson du four (1).

3. Appareil électroménager (1) selon la revendication précédente, le four (1) comprenant un élément complémentaire (i) d'alimentation électrique et de communication de données, l'élément complémentaire (i) étant complémentaire de l'élément (e) d'alimentation électrique et de l'élément (f) de communication de données du module (g) amovible, l'élément complémentaire (i) étant configuré pour raccorder électriquement l'élément (e) d'alimentation électrique du module (g) amovible à une alimentation électrique de l'appareil électroménager (1) et pour assurer une communication de données entre l'élément (f) de communication de données du module (g) amovible et une unité de contrôle et de régulation de l'appareil électroménager (1).

4. Appareil électroménager selon l'une des revendications 2 ou 3, dans lequel l'appareil électroménager est un four, de préférence un four à chaleur tournante.

5. Appareil électroménager selon l'une des revendications 2 ou 3, dans lequel l'appareil électroménager est un cuiseur électrique sous pression.

6. Procédé (100) de cuisson d'un plat avec un appareil électroménager (1) selon l'une des revendications 3 à 5, comprenant les étapes suivantes :
- Sélection (101) par un utilisateur d'un plat via une interface utilisateur, l'unité de contrôle et de régulation de l'appareil électroménager (1) étant configurée pour communiquer avec une base de données comportant une liste de plats et des programmes de cuisson associés audits plats, lesdits programmes de cuisson comportant une température de régulation, un temps de cuisson et au moins une valeur de dépassement de seuil de teneurs en composés organiques volatiles,
- Commande (102) d'une séquence de cuisson dudit plat comprenant une succession de couples de température de régulation et de temps de cuisson, la commande étant réalisée par l'unité de contrôle et de régulation de l'appareil électroménager (1), la température et/ou le temps de cuisson étant ajustés en cours de cuisson sur la base d'une détection d'une valeur de dépassement de seuils de teneurs en composés organiques volatiles détectée par le module (g) amovible en cours de cuisson du plat dans l'enceinte de cuisson de l'appareil électroménager (1).

7. Procédé (200) de calibration d'un module g amovible selon la revendication 1 pour analyser les odeurs générées dans une enceinte de cuisson de l'appareil électroménager (1), le procédé (100) comprenant les étapes suivantes :
- Sélection (201) via l'interface utilisateur d'un programme de calibration;
- Commande (202), par l'unité de contrôle et de régulation de l'appareil électroménager1, du chauffage de l'enceinte de cuisson de l'appareil électroménager1 à une température prédéterminée;
- Lorsque la température prédéterminée est atteinte dans l'enceinte de cuisson, positionnement (203) du module g amovible sur l'appareil électroménager1;
- Calibration (204) de l'élément b sensible du module g amovible sur la base d'un dépassement de seuil de teneurs en composés organiques volatiles présents dans l'enceinte de cuisson.

8. Procédé (200) de calibration selon la revendication 7, dans lequel le programme de calibration comprend une étape de préchauffage de l'appareil électroménager.

## Patentansprüche

1. Abnehmbares Modul (g) zur Analyse von Gerüchen, die in einem Garraum eines elektrischen Haushaltsgeräts (1) erzeugt werden, wobei das Modul (g) ein Gehäuse umfasst, das mit einer Einlassleitung (a) ausgestattet ist, wobei die Einlassleitung (a) in das Innere des Gehäuses mündet, wobei das Modul (g) im Inneren des Gehäuses umfasst :
- mindestens ein gasempfindliches Element (b), das so angeordnet ist, dass es auf Grenzwertüberschreitungen des Gehalts an flüchtigen organischen Verbindungen reagiert ;
- ein Saugelement (c), das so angeordnet ist, dass es ein Gas von außerhalb des Einlasskanals des Gehäuses in das Innere des Gehäuses entnimmt ;
- ein Heizelement (d) ;
- ein Stromversorgungsteil (e), das so konfiguriert ist, dass es elektrisch mit einer elektrischen Energiequelle außerhalb des Gehäuses verbunden werden kann ;
- ein Element (f) zur Datenkommunikation ;
wobei das abnehmbare Modul (g) so positioniert ist, dass es von einem Benutzer des Ofens (1) leicht zugänglich ist, und Befestigungsmittel umfasst, die dazu bestimmt sind, mit komplementären Befestigungsmitteln zusammenzuwirken, die an einer Außenfläche einer Wand des Haushaltsgeräts angeordnet sind, um das abnehmbare Modul relativ zur Wand des Haushaltsgeräts in einer Funktionsposition abnehmbar zu befestigen, in der die Einlassleitung des Gehäuses in Gasverbindung mit einem Durchgang steht, der durch die Wand des Haushaltsgeräts verläuft und in einen Abschnitt (h) des Garraums des Haushaltsgeräts (1) mündet, wobei das Gehäuse einen umschlossenen Raum definiert.

2. Haushaltsgerät (1), das mit einem abnehmbaren Modul (g) nach Anspruch 1 ausgestattet ist, wobei der Ofen (1) die komplementären Befestigungsmittel umfasst, die an der Außenfläche der Wand des Ofens (1) angeordnet sind, um mit den Befestigungsmitteln des abnehmbaren Moduls (g) zusammenzuwirken und das abnehmbare Modul (g) in einer Funktionsposition abnehmbar am Ofen (1) zu befestigen, in der die Einlassleitung des Gehäuses des abnehmbaren Moduls (g) in Gasverbindung mit einem Durchgang steht, der durch die Wand des Ofens verläuft und in einen Abschnitt des Garraums des Ofens (1) mündet.

3. Haushaltsgerät (1) nach dem vorhergehenden Anspruch, wobei der Ofen (1) ein komplementäres Element (i) zur Stromversorgung und Datenkommunikation umfasst, wobei das komplementäre Element (i) komplementär zu dem Element (e) zur Stromversorgung und dem Element (f) zur Datenkommunikation des abnehmbaren Moduls (g) ist, wobei das komplementäre Element (i) so konfiguriert ist, dass es das Element (e) zur Stromversorgung des abnehmbaren Moduls (g) elektrisch mit einer Stromversorgung des Haushaltsgeräts (1) verbindet und eine Datenkommunikation zwischen dem Element (f) zur Datenkommunikation des abnehmbaren Moduls (g) und einer Steuer- und Regeleinheit des Haushaltsgeräts (1) gewährleistet.

4. Haushaltsgerät nach einem der Ansprüche 2 oder 3, wobei das Haushaltsgerät ein Backofen, vorzugsweise ein Umluftofen, ist.

5. Haushaltsgerät nach einem der Ansprüche 2 oder 3, wobei das Haushaltsgerät ein elektrischer Druckkocher ist.

6. Verfahren (100) zum Kochen eines Gerichts mit einem Haushaltsgerät (1) nach einem der Ansprüche 3 bis 5, das die folgenden Schritte umfasst:
- Auswahl (101) eines Gerichts durch einen Benutzer über eine Benutzerschnittstelle, wobei die Steuer- und Regeleinheit des Haushaltsgeräts (1) so konfiguriert ist, dass sie mit einer Datenbank kommuniziert, die eine Liste von Gerichten und den Gerichten zugeordnete Kochprogramme umfasst, wobei die Kochprogramme eine Regeltemperatur, eine Kochzeit und mindestens einen Wert für die Überschreitung eines Schwellenwerts für den Gehalt an flüchtigen organischen Verbindungen umfassen ;
- Steuerung (102) einer Kochsequenz des Gerichts, die eine Folge von Paaren aus Regeltemperatur und Kochzeit umfasst, wobei die Steuerung durch die Steuer- und Regeleinheit des Elektrohaushaltsgeräts (1) erfolgt, wobei die Temperatur und/oder die Kochzeit während des Kochens auf der Grundlage einer Erfassung eines Werts der Überschreitung von Schwellenwerten für den Gehalt an flüchtigen organischen Verbindungen angepasst werden, der durch das abnehmbare Modul (g) während des Kochens des Gerichts im Kochraum des Elektrohaushaltsgeräts (1) erfasst wird.

7. Verfahren (200) zur Kalibrierung eines abnehmbaren Moduls g nach Anspruch 1 zur Analyse von Gerüchen, die in einem Garraum des Haushaltsgeräts 1 erzeugt werden, wobei das Verfahren (100) die folgenden Schritte umfasst:
- Auswahl (201) über die Benutzeroberfläche eines Kalibrierungsprogramms ;
- Steuerung (202) durch die Steuer- und Regeleinheit des Haushaltsgeräts 1, um den Garraum des Haushaltsgeräts 1 auf eine vorbestimmte Temperatur zu erhitzen ;
- Wenn die vorbestimmte Temperatur im Garraum erreicht ist, Positionierung (203) des abnehmbaren Moduls g auf dem Haushaltsgerät 1 ;
- Kalibrierung (204) des empfindlichen Elements b des abnehmbaren Moduls g auf der Grundlage einer Schwellenwertüberschreitung des Gehalts an flüchtigen organischen Verbindungen, die in der Garkammer vorhanden sind.

8. Verfahren (200) zur Kalibrierung nach Anspruch 7, wobei das Kalibrierungsprogramm einen Schritt zum Vorwärmen des Haushaltsgeräts umfasst.

## Claims

1. A removable module (g) for analyzing odors generated in a cooking enclosure of a household electrical appliance (1), the module (g) comprising a housing equipped with an inlet duct (a), the inlet duct (a) opening out inside the housing, the module (g) comprising, inside the housing :
- at least one gas-sensitive element (b) arranged to react to threshold levels of volatile organic compounds being exceeded;
- a suction element (c) arranged to draw a gas from outside the housing inlet duct into the housing;
- a heating element (d) ;
- a power supply element (e), configured to be electrically connected to a power source external to the housing;
- a data communication element (f);
the removable module (g) being positioned so that it is easily accessible by a user of the oven (1), and comprising fastening means designed to interact with complementary fastening means arranged on an external surface of a wall of the household appliance so as to removably fasten the removable module relative to the wall of the household appliance in an operative position in which the inlet duct of the housing is in gaseous communication with a passage provided through the wall of the household appliance and opening into a portion (h) of the cooking enclosure of the household appliance (1), the housing defining a confined space.

2. Domestic appliance (1) equipped with a removable module (g) according to claim 1, the oven (1) comprising complementary fastening means arranged on the outer surface of the wall of the oven (1) for interacting with the fastening means of the removable module (g) and removably fastening the removable module (g) to the oven (1) in an operating position in which the inlet duct of the housing of the removable module (g) is in gaseous communication with a passage provided through the wall of the oven and opening into a portion of the cooking chamber of the oven (1).

3. A domestic appliance (1) according to the preceding claim, the oven (1) comprising a complementary power supply and data communication element (i), the complementary element (i) being complementary to the power supply element (e) and data communication element (f) of the removable module (g), the complementary element (i) being configured to electrically connect the power supply element (e) of the removable module (g) to a power supply of the household electrical appliance (1) and to provide data communication between the data communication element (f) of the removable module (g) and a control and regulation unit of the household electrical appliance (1).

4. A domestic appliance according to one of claims 2 or 3, wherein the domestic appliance is an oven, preferably a fan-assisted oven.

5. A domestic appliance according to one of claims 2 or 3, wherein the domestic appliance is an electric pressure cooker.

6. A method (100) of cooking a dish with a domestic electrical appliance (1) according to one of claims 3 to 5, comprising the following steps:
- Selection (101) by a user of a dish via a user interface, the control and regulation unit of the household electrical appliance (1) being configured to communicate with a database comprising a list of dishes and cooking programs associated with said dishes, said cooking programs comprising a regulation temperature, a cooking time and at least one volatile organic compound content threshold overshoot value;
- Control (102) of a cooking sequence of said dish comprising a succession of pairs of regulation temperature and cooking time, the control being performed by the control and regulation unit of the household electrical appliance (1), the temperature and/or cooking time being adjusted during cooking on the basis of a detection of a value exceeding thresholds of volatile organic compound contents detected by the removable module (g) during cooking of the dish in the cooking chamber of the household electrical appliance (1).

7. A method (200) for calibrating a removable g-module according to claim 1 for analyzing odors generated in a cooking chamber of household appliance 1, the method (100) comprising the following steps:
- Selection (201) of a calibration program via the user interface ;
- Control (202), by the control and regulation unit of the household electrical appliance 1, of the heating of the cooking chamber of the household electrical appliance 1 to a predetermined temperature;
- When the predetermined temperature is reached in the cooking chamber, the removable g module is positioned (203) on the household appliance 1 ;
- Calibration (204) of the sensitive b element of the removable g module on the basis of exceeding the threshold of volatile organic compounds present in the cooking chamber.

8. Calibration method (200) according to claim 7, in which the calibration program comprises a step of preheating the household appliance.
